# EUROPEAN PATENT SPECIFICATION

(11) **EP 4 023 139 B1**
(45) Date of publication and mention of the grant of the patent: **08.05.2024**
(21) Application number: 21182212.7
(22) Date of filing: 29.06.2021
(51) Int. Cl.: A61B 1/05, A61B 1/00, A61B 1/06, A61B 1/12, G02B 23/24

(54) **CAMERA MODULE AND ENDOSCOPE**
KAMERAMODUL UND ENDOSKOP
MODULE DE CAMÉRA ET ENDOSCOPE

(30) Priority: 29.12.2020 CN 202011590826
(43) Date of publication of application: 06.07.2022
(73) Proprietor: Shenzhen Yateks Co., Ltd, Shenzhen, Guangdong 518000 (CN)
(72) Inventor: YAN, Jiangfan, Shenzhen, 518000 (CN); CHEN, Juhao, Shenzhen, 518000 (CN); WANG, Zhongkai, Shenzhen, 518000 (CN); GUAN, Hongquan, Shenzhen, 518000 (CN); ZOU, Haijuan, Shenzhen, 518000 (CN); ZHENG, Xiang, Shenzhen, 518000 (CN)
(74) Representative: JD&P Patent Attorneys Joanna Dargiewicz & Partners

(56) References cited:
- WO-A1-2019/154643
- US-A1- 2014 066 711
- US-A1- 2014 094 651
- US-A1- 2019 150 711

## Description

### TECHNICAL FIELD

The present disclosure relates to the field of optical technologies, and in particular to a camera module and an endoscope.

### BACKGROUND

Endoscopes are widely used in the medical field, industrial field, etc. Endoscope use environment is generally harsh, module with a year time basically have problems, and light source with a year time will decay. At present, the light source in the endoscope module on the market needs to be disassembled when replacing the whole machine, resulting in a particularly troublesome endoscope maintenance and repair.

Document US 2014/094651 A1 discloses the preamble of claim 1.

### SUMMARY OF THE DISCLOSURE

To solve the above technical problems, embodiments of the present disclosure provide a camera module and an endoscope that allow easy replacement of the light source assembly for easy maintenance.

The embodiments of the present disclosure solve the technical problems with the following technical solutions.

In a first aspect, a camera module for an endoscope comprising the features of claim 1 is provided.

The invention is defined by independent claim 1. Preferred embodiments are defined in the dependent claims.

In a second aspect, an endoscope is provided, including:
- a host; and
- a camera module according to any one of the above embodiments; wherein the camera module is further arranged with a signal line connected to the host

Compared with the prior art, the camera in the embodiments of the preset disclosure includes the housing, the light source assembly and the lens assembly; the light source assembly and the lens assembly are respectively disposed in the housing; the housing and the connection base are removably connected to each other, and the camera can be directly replaced when the light source assembly is damaged, thus making the maintenance of the endoscope more convenient, in addition to changing the optical parameters by replacing the camera, etc., which can meet different inspection needs.

### BRIEF DESCRIPTION OF THE DRAWINGS

One or more embodiments are exemplified by the accompanying drawings. These exemplified descriptions do not constitute a limitation on the embodiments. The elements with the same reference numerals in the drawings are denoted as similar elements, and the figures in the accompanying drawings do not constitute a limitation of scale unless specifically stated.
FIG. 1 is a structural schematic view of a camera module according to an embodiment of the present disclosure.
FIG. 2 is an exploded schematic view of the camera module shown in FIG. 1.
FIG. 3 is a cross-sectional view of the camera module shown in FIG. 1.
FIG. 4 is a cross-sectional view of a first connector of the camera module shown in FIG. 3.
FIG. 5 is a structural schematic view of a first circuit board and a signal line of the camera module shown in FIG 3.
FIG. 6 is an exploded schematic view of a housing of the camera module shown in FIG. 3.
FIG. 7 is a cross-sectional view of a housing and a first connector of the camera module shown in FIG. 3.
FIG. 8 is a structural schematic view of a second circuit board of the camera module shown in FIG. 3.
FIG. 9 is a structural schematic view of a second circuit board, a spring pin assembly, a light source assembly, and a lens assembly of the camera module shown in FIG. 3.
FIG. 10 is a cross-sectional view of a camera module according to another embodiment of the present disclosure.
FIG. 11 is a structural schematic view of a housing of the camera module shown in FIG 10.
FIG. 12 is a structural schematic view of a second circuit board, a light source assembly, and a lens assembly of the camera module shown in FIG. 10.

### DETAILED DESCRIPTION

To facilitate the understanding of the present disclosure, the present disclosure will be described in more detail below with reference to the accompanying drawings and specific embodiments. It should be noted that when an element is expressed as being "fixed to" another element it may be directly on the other element, or there may be one or more elements in between. When an element is expressed as being "connected to" another element, it may be directly connected to the other element, or there may be one or more intervening elements in between. The terms "vertical", "horizontal", "left", "right", "inner", "outer" and similar expressions used in this specification are for illustrative purposes only.

Unless otherwise defined, all technical and scientific terms used in this specification have the same meaning as commonly understood by those skilled in the technical field of the present disclosure. The terms used in the description of the present disclosure are only for the purpose of describing specific embodiments and not to limit the present disclosure. The term "and/or" used in this specification includes any and all combinations of one or more related listed items.

In addition, the technical features involved in the different embodiments of the present disclosure described below can be combined with each other as long as they do not conflict with each other.

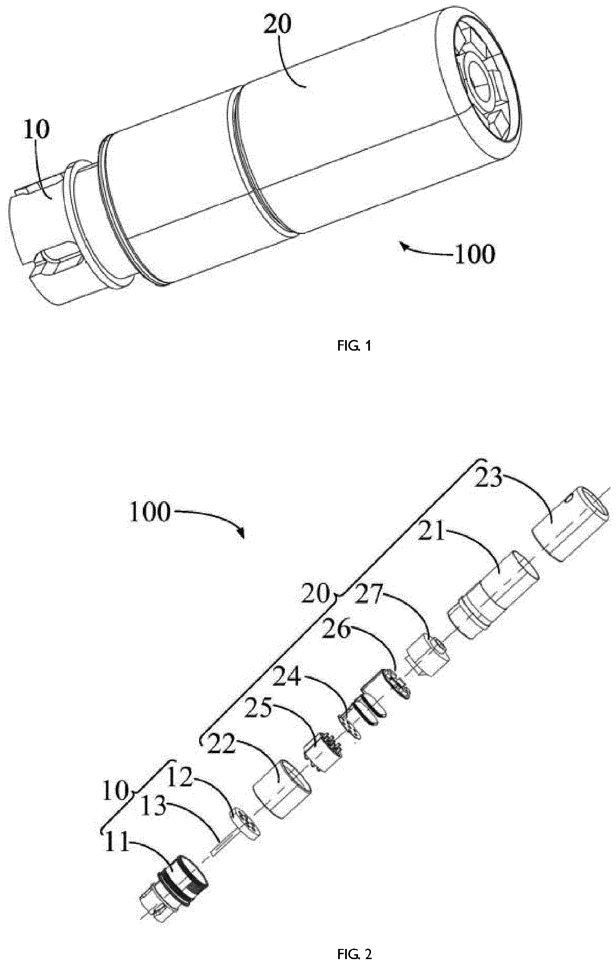

Referring to FIG. 1, an embodiment of the present disclosure provides a camera module 100, which includes a connecting base 10 and at least one camera 20. The connecting base 10 and the at least one camera 20 are detachably connected. The number of the cameras 20 may be multiple, and the cameras 20 may be respectively configured with different parameters. The cameras 20 may be replaced and installed on the connecting base 10 to change the depth of field, viewing angle, endoscope viewing direction and other parameters of the camera module 100. The camera 20 may be a direct-view camera, a side-view camera, a direct-view camera and a side-view dual camera, etc. In the embodiment, a direct-view camera is taken as an example for description.

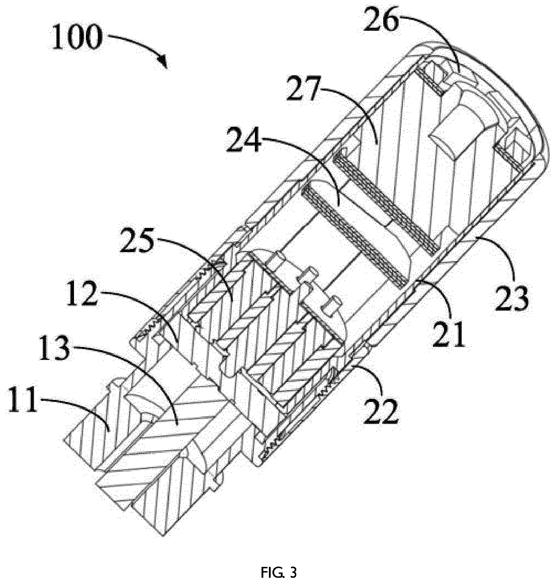

Referring to FIGS. 2 and 3 together, the connecting base 10 may include a first connecting member 11, a first circuit board 12, and a signal line 13. The first circuit board 12 is installed in the first connecting member 11. The signal line 13 is connected to the first circuit board 12. An end of the first connecting member 11 is configured to connect to the camera 20, and the other end of the first connecting member 11 is connected to an external pipe.

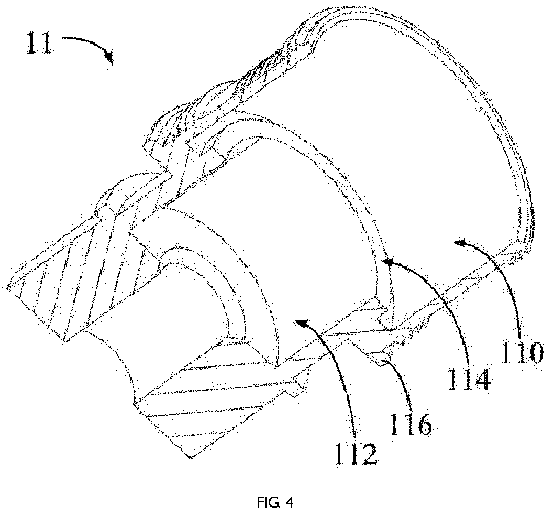

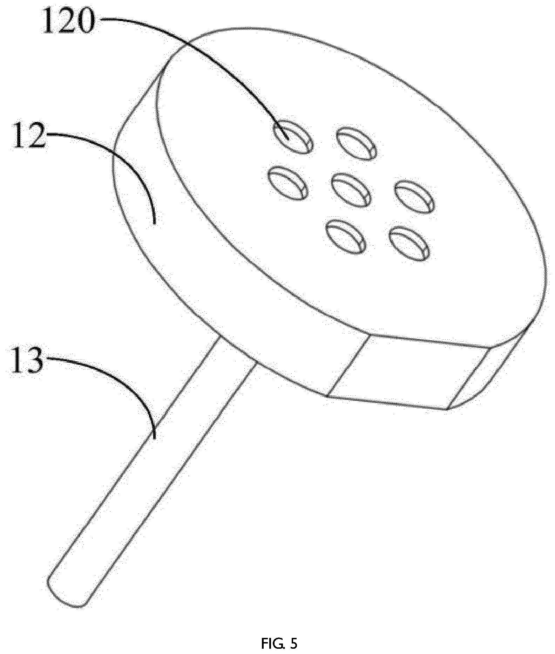

Referring to FIGS. 3 to 5 together, the first connecting member 11 may be a hollow round tube. An inside of the first connecting member 11 defines a stepped hole including a first receiving hole 110 and a second receiving hole 112. The first receiving hole 110 and the second receiving hole 112 are in communication. The diameter of the first receiving hole 110 is greater than the diameter of the second receiving hole 112. A stepped surface 114 is formed between the first receiving hole 110 and the second receiving hole 112. The first receiving hole 110 is disposed facing the camera 20, the first circuit board 12 is disposed in the first receiving hole 110, and the first circuit board 12 abuts the stepped surface 114. The signal line 13 is connected to a side of the first circuit board 12 facing away from the camera 20. The signal line 13 passes through the second receiving hole 112 to be connected to an external host The first connecting member 11 is arranged with a positioning outer ring 116. The positioning outer ring 116 protrudes from an outer wall of the first connecting member 11. The positioning outer ring 116 is configured to position the camera 20. An end of the first connecting member 11 facing the camera 20 is further arranged with an external thread configured to connect with the camera 20.

It can be understood that in some other embodiments, the shape of the first connecting member 11 may be set according to actual needs, such as a square tube, a triangular tube, an elliptical tube, etc., just to be able to meet the installation of the first circuit board 12 and the signal line 13.

The first circuit board 12 may be plate-shaped. A side of the first circuit board 12 facing away from the camera 20 abuts on the stepped surface 114, and the other side of the first circuit board 12 is arranged with a plurality of metal contacts 120 configured for electrical connection with the camera 20. The shape of the first circuit board 12 matches the shape of the first receiving hole 110, and an outer edge of the first circuit board 12 abuts an inner wall of the first receiving hole 110 to facilitate installation of the first circuit board 12.

Referring to FIG. 2 and 3 again, the camera 20 may include a housing, a second circuit board 24, a spring pin assembly 25, a light source assembly 26, and a lens assembly 27. The second circuit board 24, the spring pin assembly 25, the light source assembly 26 and the lens assembly 27 are respectively installed in the housing. The housing includes a fixing member 21, a second connecting member 22 and a casing 23. The second connecting member 22 and the casing 23 are respectively connected to the fixing member 21. The spring pin assembly 25 is connected to an end of the second circuit board 24. The light source assembly 26 is connected to the other end of the second circuit board 24. The lens assembly 27 is disposed between the spring pin assembly 25 and the light source assembly 26, and is connected to the second circuit board 24.

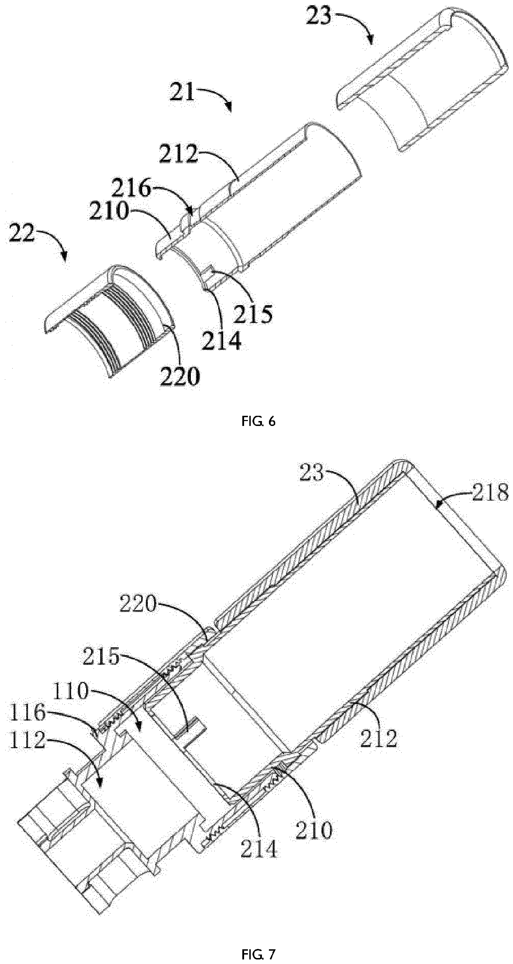

Referring to FIG 3, FIG 6, and FIG 7 together, the fixing member 21 may be a hollow round tube. An inside of the fixing member 21 defines an installation cavity. The second circuit board 24, the spring pin assembly 25, the light source assembly 26 and the lens assembly 27 are respectively installed in the installation cavity. The fixing member 21 may be a stepped shaft and include a first fixing portion 210 and a second fixing portion 212. The first fixing portion 210 and the second fixing portion 212 are connected. The diameter of the first fixing portion 210 is less than the diameter of the second fixing portion 212. The first connecting member 11 is sleeved on the first fixing portion 210. The second connecting member 22 and the casing 23 are sleeved on the second fixing portion 212 respectively. The first fixing portion 210 of the fixing member 21 is disposed in the first receiving hole 110 of the first connecting member 11, and an end of the first fixing portion 210 facing away from the second fixing portion 212 abuts the first circuit board 12.

The end of the first fixing portion 210 facing away from the second fixing portion 212 is further arranged with a positioning inner ring 214. The positioning inner ring 214 protrudes from an inner wall of the first fixing portion 210 toward an inner cavity. The positioning inner ring 210 is configured to position the spring pin assembly 25. The inner wall of the first fixing portion 210 is further arranged with a limiting block 215, the limiting block 215 extending from the positioning inner ring 214 in a direction away from the second fixing portion 212. The limiting block 215 is configured to limit the position of the spring pin assembly 25.

An end of the second fixing portion 212 facing the first fixing portion 210 further defines a clamping groove 216 configured to connect with the second connecting member 22. A groove wall at an end of the clamping groove 216 facing the first fixing portion 212 is an abutting wall configured to limit the position of the second connecting member 22. An end of the second fixing portion 212 facing away from the first fixing portion 210 defines a light-transmitting window 218 communicating the installation cavity with an outside.

The second connecting member 22 may be a hollow round tube, and an end of the second connecting member 22 facing the connecting base 10 is arranged with an internal thread. The second connecting member 22 is sleeved on the first connecting member 11. The internal thread of the second connecting member 22 matches the external thread of the first connecting member 11, such that the second connecting member 22 is fixed to the first connecting member 11. An end of the second connecting member 22 facing away from the connecting base 10 is arranged with a clamping portion 220, and the clamping portion 220 protrudes from an inner wall of the second connecting member 22. An end of the second connecting member 22 facing away from the connecting base 10 is sleeved on the second fixing portion 212 of the fixing member 21. The clamping portion 220 is disposed in the clamping groove 216. An end of the clamping portion 220 facing the first connecting member 11 abuts against the abutting wall.

It can be understood that, in some other embodiments, the connection mode of the second connecting member 22 and the first connecting member 11 may be set according to actual needs, such as a snap connection, a fastener connection, a key connection, etc., just to be able to meet the detachable connection requirement between the first connecting member 11 and the second connecting member 22.

The casing 23 may be a hollow round tube. The casing 23 is sleeved on an end of the second fixing portion 212 facing away from the first fixing portion 210. The casing 23 abuts against an end of the clamping portion 220 of the second connecting member 22 away from the first connecting member 11, such that the second connecting member 22 is axially fixed. A window corresponding to the light-transmitting window 218 is defined at an end of the casing 23 facing away from the second connecting member 22 to ensure that the light-transmitting window 218 communicates with the outside.

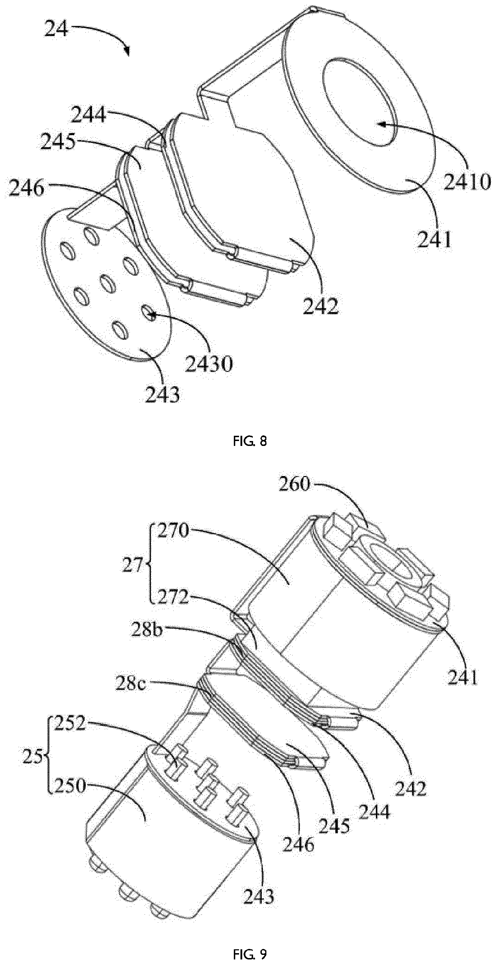

Referring to FIGS. 8 and 9 together, the second circuit board 24 may be a flexible circuit board, and the second circuit board 24 may include a first board portion 241, a second board portion 242, and a third board portion 243 connected in sequence. The first board portion 241, the second board portion 242, and the third board portion 243 are sequentially arranged along an axial direction of the fixing member 21. The first board portion 241, the second board portion 242 and the third board portion 243 are spaced apart The first board portion 241 is disposed at an end of the second fixing portion 212 facing away from the first fixing portion 210, and the third board portion 243 is disposed on the other end of the second fixing portion 212.

The first board portion 241 may have an annular plate shape and define a through hole 2410. The light source assembly 26 is arranged on a side of the first board portion 241 facing away from the second board portion 242 and faces the light-transmitting window 218. The emitted light can be transmitted through the light-transmitting window 218 to the outside. The lens assembly 27 is disposed between the first board portion 241 and the second board portion 242, and two ends of the lens assembly 27 are respectively connected to the first board portion 241 and the second board portion 242. An end of the lens assembly 27 passes through the through hole 2410. The third board portion 243 defines a plurality of connecting holes 2430 configured to connect with the spring pin assembly 25.

In some embodiments, the first board portion 241 is arranged with a heat sink 28a on a side facing away from the light source assembly 26, the heat sink 28a abuts against the lens assembly 27, and the heat sink 28a abuts against an inner wall of the fixing member 21. The heat sink 28a can conduct the heat generated during the operation of the light source assembly 26 and the lens assembly 27 to the housing. The second board portion 242 is further arranged with a heat sink 28b on a side facing away from the lens assembly 27, the heat sink 28b abuts against the inner wall of the fixing member 21, and the heat sink 28b can conduct the heat generated during the operation of the lens assembly 27 to the housing.

In some embodiments, the second circuit board 24 may further include a fourth board portion 244, a fifth board portion 245, and a sixth board portion 246 that are sequentially connected. The fourth board portion 244, the fifth board portion 245, and the sixth board portion 246 are sequentially arranged at intervals along the axial direction of the fixing member 21. The fourth board portion 244, the fifth board portion 245, and the sixth board portion 246 are disposed between the second board portion 242 and the third board portion 243. The fourth board portion 244 is connected to the second board portion 242, and the sixth board portion 246 is connected to the third board portion 243. The heat sink 28b is disposed between the second board portion 242 and the fourth board portion 244. A heat sink 28c is further arranged between the fifth board portion 245 and the sixth board portion 246. The heat sink 28c abuts against the inner wall of the fixing member 21 for further dissipating heat for the light source assembly 26 and the lens assembly 27.

In some embodiments, the installation cavity of the fixing member 21 is potted with thermally conductive glue, such that the second circuit board 24 and the fixing member 21 are fixedly connected, and the heat conduction between the second circuit board 24 and the fixing member 21 is increased, which can better dissipate heat for the light source assembly 26 and the lens assembly 27.

The spring pin assembly 25 may include a spring pin base 250 and a plurality of spring pins 252. The plurality of spring pins 252 are installed on the spring pin base 250. A plurality of installation through holes (not shown) are defined on the spring pin base 250. The installation through holes penetrate through two ends of the spring pin base 250 in an axial direction. Each spring pin 252 is installed in a corresponding installation through hole.

The spring pin base 250 is disposed in the first fixing portion 210. An end of the spring pin base 250 abuts the positioning inner ring 214, and the other end of the spring pin base 250 abuts the third board portion 243, such that the spring pin base 250 is fixed along the axial direction of the fixing member 21. An outer wall of the spring pin base 250 defines a limiting groove 2500. The shape of the limiting groove 2500 matches the shape of the limiting block 215, and the limiting block 215 is clamped in the limiting slot 2500, such that the spring pin base 250 is fixed along the axial direction of the fixing member 21.

An end of each spring pin 252 is disposed in one of the plurality of connecting holes 2430 of the third board portion 234, and each spring pin 252 corresponds to one of the connecting holes 2430. The plurality of spring pins 252 are welded and fixed to the third board portion 234. The other end of each spring pin 252 abuts against the plurality of metal contacts 120 of the first circuit board 12, and each spring pin 252 corresponds to one of the metal contacts 120. The spring pins 252 can enable the electrical connection between the first circuit board 12 and the second circuit board 23, and can conduct heat on the second circuit board 23 to the first circuit board 12 for heat dissipation.

The light source assembly 26 may include a plurality of lamp beads 260 annularly distributed on the first board portion 241. The plurality of lamp beads 260 are welded to the first board portion 241 such that the plurality of lamp beads 260 are electrically connected to the second circuit board 24. Light-emitting ends of the plurality of lamp beads 260 face the light-transmitting window 218, and the plurality of lamp beads 260 can emit light toward the outside of the camera 20. In the embodiments, each lamp bead may be a light-emitting diode (LED), and the LED can have any light source wavelength band to meet different requirements.

The lens assembly 27 may include an optical lens 270 and an image sensor 272. An end of the optical lens 270 is connected to the image sensor 272. The other end of the optical lens 270 passes through the through hole 2410 to communicate with the outside. The optical lens 270 is configured to refract and filter light The image sensor 272 is soldered to the second board portion 242 such that the image sensor 272 is electrically connected to the second circuit board 24. The external light enters through the light-transmitting window 218 and is refracted by the optical lens 270 and transmitted to the image sensor 272. The image sensor 272 generates image information after processing.

The assembly process of the camera module 100 will be briefly described below in conjunction with the drawings.

When the connecting base 10 is assembled, the first circuit board 12 and the signal line 13 are welded first the signal line 13 passes through the first receiving hole 110 and the second receiving hole 112 in sequence, and the first circuit board 12 is disposed in the first receiving hole 110 and abuts the stepped surface 114.

When the camera 20 is assembled, the second circuit board 24 is bent, and the spring pin assembly 25, the light source assembly 26, and the lens assembly 27 are respectively welded on the second circuit board 24. The second circuit board 24, the spring pin assembly 25, the light source assembly 26, and the lens assembly 27 are installed in the fixing member 21 after assembled. The second connecting member 22 is sleeved on the fixing member 21, and the casing 23 is sleeved on the fixing member 21 and abuts against the second connecting member 22.

When the connecting base 10 is assembled with the camera 20, the second connecting member 22 is sleeved on the first connecting member 11, the second connecting member 22 is rotated to screw with the first connecting member 11 until the second connecting member 22 abuts against the positioning outer ring 116, and the spring pins 252 abut against the metal contacts 120.

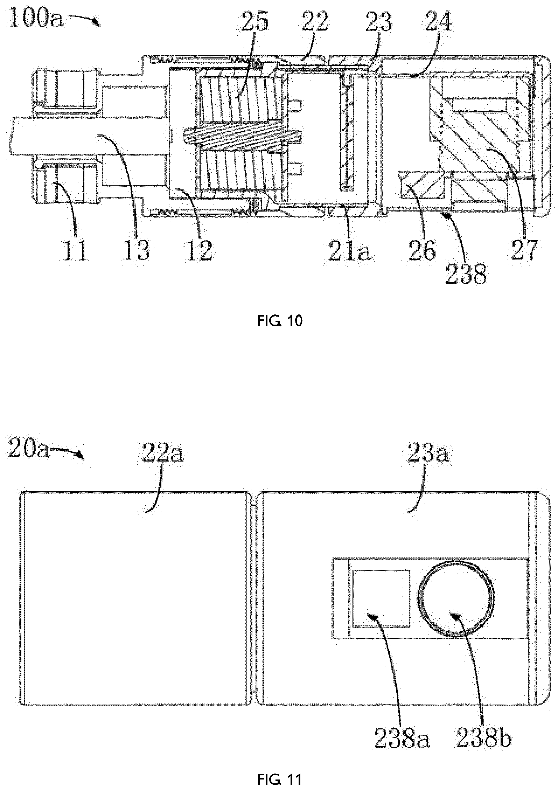

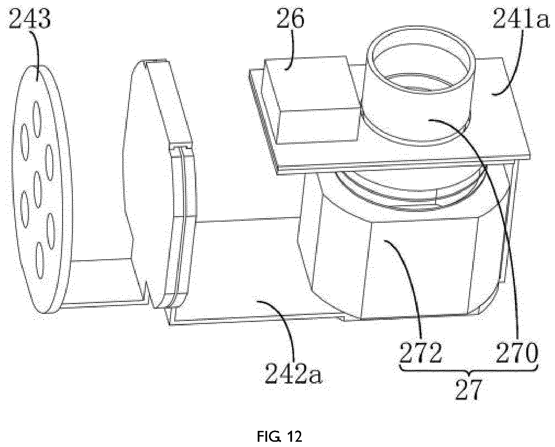

Referring to FIGS. 10 to 12, another embodiment of the present disclosure provides a camera module 100a which is basically the same as the camera module 100 in the above embodiments, except that the camera 20a of the camera module 100a is a side-view camera. Specifically, the end of the casing 23a facing away from the second connecting member 22a extends axially out of the fixing member 21a, and the inner cavity of the casing 23a is in communication with the installation cavity of the fixing member 21a. The light-transmitting window 238 is defined on a side wall of the end of the casing 23a facing away from the second connecting member 22a. The light-transmitting window 238 communicates the inner cavity of the casing 23a with the outside, and the end of the casing 23a away from the second connecting member 22a is not in communication with the outside. The light-transmitting window 238 includes a first light-transmitting window 238a and a second light-transmitting window 238b distributed along the axial direction of the casing 23a. The second circuit board 24a extends from the installation cavity to the inner cavity of the casing 23a, the first board portion 241a and the second board portion 242a are disposed in the inner cavity of the casing 23a, and the first board portion 241a and the second board portion 242a are arranged along a radial direction of the casing 23a. The light source assembly 26 is arranged on the side of the first board portion 241a facing the light-transmitting window 238, and the position of the light source assembly 26 corresponds to the position of the first light-transmitting window 238a. The lens assembly 27 is disposed between the first board portion 241a and the second board portion 242a, the optical lens 270 is disposed on a side of the light source assembly 26 along the axial direction of the casing 23a, and the position of the optical lens 270 corresponds to the position of the second light-transmitting window 238b. Based on the above method, the camera module 100a provided in this embodiment can realize lateral shooting and meet the shooting requirements in different environments.

Another embodiment of the present disclosure also provides a camera module 100b which is basically the same as the camera module 100 in the foregoing embodiment, except that the camera of the camera module 100b is a direct-view and a side-view dual camera. Specifically, the end of the second fixing portion away from the first fixing portion defines a light-transmitting window, and the side wall of the second fixing portion defines another light-transmitting window. The casing defines two windows corresponding to the two light-transmitting windows, such that the two light-transmitting windows are respectively connected to the outside. The second circuit board is arranged with two light source assemblies and two lens assemblies, each light source assembly faces a corresponding one of the light-transmitting windows, and each lens assembly faces a corresponding one of the light-transmitting windows. Based on the above method, the camera module 100a provided in this embodiment can realize simultaneous vertical and lateral shooting, which meets the shooting requirements in different environments.

Another embodiment of the present disclosure provides an endoscope. The endoscope includes a host and the camera module described in any of the above embodiments. The signal line of the camera module is connected to the host to transmit image information of the image sensor to the host

Finally, it should be noted that the above embodiments are only to illustrate the technical solutions of the present disclosure, not to limit them; under the idea of the present disclosure, the technical features of the above embodiments or different embodiments can also becombined, all without departing from the scope of the invention as defined by the appended claims. For the sake of brevity, they are not provided in details; although the present disclosure has been described in detail with reference to the foregoing embodiments, those skilled in the art should understand that: they can still modify the technical solutions recorded in the foregoing embodiments, without departing from the scope of the invention as defined by the appended claims.

## Claims

1. A camera module (100) for an endoscope, comprising:
a connecting base (10) and a camera (20);
the connecting base (10) comprises a first connecting member (11) and a first circuit board (12); wherein the first circuit board (12) is installed in the first connecting member (11); and
the camera (20) comprises a housing, a second circuit board (24), a light source assembly (26), and a lens assembly (27); wherein the second circuit board (24), the light source assembly (26), and the lens assembly (27) are respectively installed in the housing; the housing is removably connected to the first connecting member (11); the first circuit board (12) and the second circuit board (24) are electrically connected to each other;
**characterised in that**
an inside of the first connecting member (11) defines a stepped hole including a first receiving hole (110) and a second receiving hole (112); the first receiving hole (110) and the second receiving hole (112) are in communication; the diameter of the first receiving hole (110) is greater than the diameter of the second receiving hole (112);a stepped surface (114) is formed between the first receiving hole (110) and the second receiving hole (112); the first receiving hole (110) is disposed facing the camera (20), the first circuit board (12) is disposed in the first receiving hole (110), and the first circuit board (12) abuts the stepped surface (114).

2. The camera module (100) for an endoscope according to claim 1, wherein the second circuit board (24) comprises a first board portion (241), a second board portion (242), and a third board portion (243) connected in sequence; the first board portion (241) is disposed at an end of the housing facing away from the first connecting member (11), and the third board portion (243) is disposed on the other end of the housing; the light source assembly (26) is disposed on a side of the first board portion (241) facing away from the second board portion (242); the lens assembly (27) is disposed between the first board portion (241) and the second board portion (242).

3. The camera module (100) for an endoscope according to claim 2, wherein the first board portion (241) is arranged with a heat sink (28a) on a side facing away from the light source assembly (26), the heat sink (28a) abutting against the lens assembly (27); and/or
the second board portion (242) is arranged with a heat sink (28b) on a side facing away from the lens assembly, the heat sink (28b) abutting against an inner wall of the housing.

4. The camera module (100) for an endoscope according to claim 2, wherein the light source assembly (26) comprises a plurality of lamp beads (260) annularly distributed on the first board portion (241) the housing defines a light-transmitting window (218), and the plurality of lamp beads (260) face the light-transmitting window (218).

5. The camera module (100) for an endoscope according to claim 4, wherein the lens assembly (27) comprises an optical lens (270) and an image sensor (272); the image sensor (272) is disposed at the second board portion (242); an end of the optical lens (270) is connected to the image sensor (272); the first board portion (241) defines a through hole (2410); the other end of the optical lens (270) passes through the through hole (2410) and faces the light-transmitting window (218).

6. The camera module (100) for an endoscope according to any one of claims 1-5, wherein the housing comprises a fixing member (21) and a second connecting member (22); an end of the second connecting member (22) is sleeved on the fixing member (21), and the other end of the second connecting member (22) is sleeved on the first connecting member (11 );
an inside of the fixing member (21) defines an installation cavity, and the second circuit board (24) is installed in the installation cavity.

7. The camera module (100) for an endoscope according to claim 6, wherein the fixing member (21) defines a clamping groove (216), and an end of the second connecting member (22) facing away from the first connecting member (11) is arranged with a clamping portion (220); the clamping portion (220) is disposed in the clamping groove (216); an end of the clamping portion (220) facing the first connecting member (11) abuts against a wall of the clamping groove (216);
the housing further comprises a casing (23); the casing (23) is sleeved on an end of the fixing member (21) facing away from the first connecting member (11), and the casing (23) abuts against the other end of the clamping portion (220).

8. The camera module (100) for an endoscope according to claim 1, further comprising a spring pin assembly (25) disposed in the housing and connected between the first circuit board (12) and the second circuit board (24).

9. The camera module (100) for an endoscope according to claim 8, wherein the spring pin assembly (25) comprises a spring pin base (250) and a plurality of spring pins (252); the plurality of spring pins (252) are installed on the spring pin base (250);
the first circuit board (12) is arranged with a plurality of metal contacts (120); the plurality of spring pins (252) abut against the plurality of metal contacts (120).

10. An endoscope, **characterized by** comprising:
a host; and
a camera module (100) according to any one of claims 1-9; wherein the camera module (100) is further arranged with a signal line (13) connected to the host.

## Patentansprüche

1. Kameramodul (100) für ein Endoskop, umfassend: eine Verbindungsbasis (10) und eine Kamera (20);
wobei die Verbindungsbasis (10) ein erstes Verbindungselement (11) und eine erste Leiterplatte (12) umfasst; wobei die erste Leiterplatte (12) in dem ersten Verbindungselement (11) installiert ist; und
wobei die Kamera (20) ein Gehäuse, eine zweite Leiterplatte (24), eine Lichtquellenbaugruppe (26) und eine Linsenbaugruppe (27) umfasst; wobei die zweite Leiterplatte (24), die Lichtquellenbaugruppe (26) und die Linsenbaugruppe (27) jeweils in dem Gehäuse installiert sind; wobei das Gehäuse lösbar mit dem ersten Verbindungselement (11) verbunden ist; wobei die erste Leiterplatte (12) und die zweite Leiterplatte (24) elektrisch miteinander verbunden sind;
**dadurch gekennzeichnet, dass** eine Innenseite des ersten Verbindungselements (11) ein gestuftes Loch definiert, das ein erstes Aufnahmeloch (110) und ein zweites Aufnahmeloch (112) umfasst; wobei das erste Aufnahmeloch (110) und das zweite Aufnahmeloch (112) in Verbindung stehen; wobei der Durchmesser des ersten Aufnahmelochs (110) größer als der Durchmesser des zweiten Aufnahmelochs (112) ist; wobei eine abgestufte Oberfläche (114) zwischen dem ersten Aufnahmeloch (110) und dem zweiten Aufnahmeloch (112) ausgebildet ist; wobei das erste Aufnahmeloch (110) der Kamera (20) zugewandt angeordnet ist, wobei die erste Leiterplatte (12) in dem ersten Aufnahmeloch (110) angeordnet ist und die erste Leiterplatte (12) an der abgestuften Oberfläche (114) anliegt.

2. Kameramodul (100) für ein Endoskop nach Anspruch 1, wobei die zweite Leiterplatte (24) einen ersten Plattenabschnitt (241), einen zweiten Plattenabschnitt (242) und einen dritten Plattenabschnitt (243) umfasst, die hintereinander geschaltet sind; wobei der erste Plattenabschnitt (241) an einem Ende des Gehäuses angeordnet ist, das von dem ersten Verbindungselement (11) abgewandt ist, wobei der dritte Plattenabschnitt (243) an dem anderen Ende des Gehäuses angeordnet ist; wobei die Lichtquellenbaugruppe (26) auf einer Seite des ersten Plattenabschnitts (241) angeordnet ist, die von dem zweiten Plattenabschnitt (242) abgewandt ist; wobei die Linsenbaugruppe (27) zwischen dem ersten Plattenabschnitt (241) und dem zweiten Plattenabschnitt (242) angeordnet ist.

3. Kameramodul (100) für ein Endoskop nach Anspruch 2, wobei der erste Plattenabschnitt (241) mit einem Kühlkörper (28a) auf einer von der Lichtquellenbaugruppe (26) abgewandten Seite angeordnet ist, wobei der Kühlkörper (28a) an der Linsenbaugruppe (27) anliegt; und/oder wobei der zweite Plattenabschnitt (242) mit einem Kühlkörper (28b) auf einer von der Lichtquellenbaugruppe abgewandten Seite angeordnet ist, wobei der Kühlkörper (28b) an einer Innenwand des Gehäuses anliegt.

4. Kameramodul (100) für ein Endoskop nach Anspruch 2, wobei die Lichtquellenbaugruppe (26) eine Vielzahl von Lampenkugeln (260) umfasst, die ringförmig auf dem ersten Plattenabschnitt (241) verteilt sind, wobei das Gehäuse ein lichtdurchlässiges Fenster (218) definiert und die Vielzahl von Lampenkugeln (260) dem lichtdurchlässigen Fenster (218) zugewandt ist.

5. Kameramodul (100) für ein Endoskop nach Anspruch 4, wobei die Linsenbaugruppe (27) eine optische Linse (270) und einen Bildsensor (272) umfasst; wobei der Bildsensor (272) an dem zweiten Plattenabschnitt (242) angeordnet ist; wobei ein Ende der optischen Linse (270) mit dem Bildsensor (272) verbunden ist; wobei der erste Plattenabschnitt (241) ein Durchgangsloch (2410) definiert, wobei das andere Ende der optischen Linse (270) durch das Durchgangsloch (2410) hindurchgeht und dem lichtdurchlässigen Fenster (218) zugewandt ist.

6. Kameramodul (100) für ein Endoskop nach einem der Ansprüche 1 bis 5, wobei das Gehäuse ein Befestigungselement (21) und ein zweites Verbindungselement (22) umfasst, wobei ein Ende des zweiten Verbindungselements (22) auf das Befestigungselement (21) aufgeschoben ist und das andere Ende des zweiten Verbindungselements (22) auf das erste Verbindungselement (11) aufgeschoben ist; und
wobei eine Innenseite des Befestigungselements (21) einen Installationshohlraum definiert, und wobei die zweite Leiterplatte (24) in dem Installationshohlraum installiert ist.

7. Kameramodul (100) für ein Endoskop nach Anspruch 6, wobei das Befestigungselement (21) eine Klemmnut (216) definiert, wobei ein Ende des zweiten Verbindungselements (22), das von dem ersten Verbindungselement (11) abgewandt ist, mit einem Klemmabschnitt (220) versehen ist; wobei der Klemmabschnitt (220) in der Klemmnut (216) angeordnet ist; wobei ein Ende des Klemmabschnitts (220), das dem ersten Verbindungselement (11) zugewandt ist, an einer Wand der Klemmnut (216) anliegt; und
wobei das Gehäuse ferner eine Ummantelung (23) umfasst; wobei die Ummantelung (23) auf einem Ende des Befestigungselements (21), das von dem ersten Verbindungselement (11) abgewandt ist, aufgeschoben ist, und wobei die Ummantelung (23) am anderen Ende des Klemmabschnitts (220) anliegt.

8. Kameramodul (100) für ein Endoskop nach Anspruch 1, ferner umfassend eine Federstiftanordnung (25), die im Gehäuse angeordnet und zwischen der ersten Leiterplatte (12) und der zweiten Leiterplatte (24) verbunden ist.

9. Kameramodul (100) für ein Endoskop nach Anspruch 8, wobei die Federstiftanordnung (25) eine Federstiftbasis (250) und mehrere Federstifte (252) umfasst, wobei die mehreren Federstifte (252) auf der Federstiftbasis (250) installiert sind;
wobei die erste Leiterplatte (12) mit einer Vielzahl von Metallkontakten (120) versehen ist, und wobei die Vielzahl von Federstiften (252) an der Vielzahl von Metallkontakten (120) anliegen.

10. Endoskop, **dadurch gekennzeichnet, dass** es umfasst:
einen Host; und
ein Kameramodul (100) nach einem der Ansprüche 1 bis 9, wobei das Kameramodul (100) ferner mit einer mit dem Host verbundenen Signalleitung (13) versehen ist.

## Revendications

1. Module de caméra (100) pour un endoscope, comprenant :
une base de connexion (10) et une caméra (20),
la base de connexion (10) comprend un premier membre de connexion (11) et une première carte de circuit imprimée (12) ; dans lequel, la première carte de circuit imprimée (12) est installée dans le premier membre de connexion (11) ; et
la caméra (20) comprend un boîtier, une deuxième carte de circuit imprimée (24), un ensemble de source lumineuse (26), et un ensemble de lentille (27) ; dans lequel, la deuxième carte de circuit imprimée (24), l'ensemble de source lumineuse (26) et l'ensemble de lentille (27) sont installés dans le boîtier respectivement ; le boîtier est relié amovible au premier membre de connexion (11) ; la première carte de circuit imprimée (12) et la deuxième carte de circuit imprimée (24) sont reliées électriquement entre elles,
**caractérisé en ce que**
un intérieur du premier membre de connexion (11) définit un orifice étagé comprenant un premier orifice de réception (110) et un deuxième orifice de réception (112), le premier orifice de réception (110) et le deuxième orifice de réception (112) sont en communication, le diamètre du premier orifice de réception (110) est supérieur à celui du deuxième orifice de réception (112) ; une surface étagée (114) est formée entre le premier orifice de réception (110) et le deuxième orifice de réception (112), le premier orifice de réception (110) est disposé en face de la caméra (20), la première carte de circuit imprimée (12) est disposée dans le premier orifice de réception (110), et la première carte de circuit imprimée (12) s'appuie contre la surface étagée (114).

2. Module de caméra (100) pour un endoscope selon la revendication 1, dans lequel la deuxième carte de circuit imprimée (24) comprend une première portion de panneau (241), une deuxième portion de panneau (242) et une troisième portion de panneau (243) reliées en séquence ; la première portion de panneau (241) est disposée à une extrémité du boîtier opposée au premier membre de connexion (11), et la troisième portion de panneau (243) est disposée à une autre extrémité du boîtier ;
l'ensemble de source lumineuse (26) est disposé à un côté de la première portion de panneau (241) opposé à la deuxième portion de panneau (242) ; l'ensemble de lentille (27) est disposé entre la première portion de panneau (241) et la deuxième portion de panneau (242).

3. Module de caméra (100) pour un endoscope selon la revendication 2, dans lequel la première portion de panneau (241) est agencée avec un dissipateur thermique (28a) sur un côté opposé à l'ensemble de source lumineuse (26), le dissipateur thermique (28a) s'appuyant contre l'ensemble de lentille (27) ; et/ou
la deuxième portion de panneau (242) est agencée avec un dissipateur thermique (28b) sur un côté opposé à l'ensemble de lentille, le dissipateur thermique (28b) s'appuyant contre une paroi interne du boîtier.

4. Module de caméra (100) pour un endoscope selon la revendication 2, dans lequel l'ensemble de source lumineuse (26) comprend une pluralité de billes de lampe (260) réparties de manière annulaire sur la première portion de panneau (241) ; le boîtier définit une fenêtre de transmission de lumière (218), et la pluralité de billes de lampe (260) font face à la fenêtre de transmission de lumière (218).

5. Module de caméra (100) pour un endoscope selon la revendication 4, dans lequel l'ensemble de lentille (27) comprend une lentille optique (270) et un capteur d'image (272) ; le capteur d'image (272) est disposé à la deuxième portion de panneau (242) ; une extrémité de la lentille optique (270) est reliée au capteur d'image (272) ;
la première portion de panneau (241) définit un orifice traversant (2410) ; l'autre extrémité de la lentille optique (270) traverse l'orifice traversant (2410) et fait face à la fenêtre de transmission de lumière (218).

6. Module de caméra (100) pour un endoscope selon une quelconque des revendications 1 à 5, dans lequel, le boîtier comprend un membre de fixation (21) et un deuxième membre de connexion (22) ; une extrémité du deuxième membre de connexion (22) est emmanchée sur le membre de fixation (21), et l'autre extrémité du deuxième membre de connexion (22) est emmanchée sur le premier membre de connexion (11) ;
un intérieur du membre de fixation (21) définit une cavité d'installation, et la deuxième carte de circuit imprimée (24) est installée dans la cavité d'installation.

7. Module de caméra (100) pour un endoscope selon la revendication 6, dans lequel le membre de fixation (21) définit une rainure de serrage (216), et une extrémité du deuxième membre de connexion (22) opposée au premier membre de connexion (11) est agencée avec une portion de serrage (220) ; la portion de serrage (220) est disposée dans la rainure de serrage (216) ; une extrémité de la portion de serrage (220) en face au premier membre de connexion (11) s'appuie contre une paroi de la rainure de serrage (216) ;
le boîtier comprend en outre une enveloppe (23) ; l'enveloppe (23) est emmanchée sur une extrémité du membre de fixation (21) opposée au premier membre de connexion (11), et l'enveloppe (23) s'appuie contre l'autre extrémité de la portion de serrage (220).

8. Module de caméra (100) pour un endoscope selon la revendication 1, comprenant en outre un ensemble de broche à ressort (25) disposé dans le boîtier et relié entre la première carte de circuit imprimée (12) et la deuxième carte de circuit imprimée (24).

9. Module de caméra (100) pour un endoscope selon la revendication 8, dans lequel l'ensemble de broche à ressort (25) comprend une base de broche à ressort (250) et une pluralité de broches à ressort (252) ; la pluralité de broches à ressort (252) sont installées sur la base de broche à ressort (250) ;
la première carte de circuit imprimée (12) est agencée avec une pluralité de contacts métalliques (120) ; la pluralité de broches à ressort (252) s'appuient contre la pluralité de contacts métalliques (120).

10. Un endoscope, **caractérisé par** comprenant :
un hôte ; et
un module de caméra (100) selon une quelconque des revendications 1 à 9 ; dans lequel, le module de caméra (100) est en outre agencé avec une ligne de signal (13) reliée au hôte.
